(19) Europäisches Patentamt

European Patent Office

Office européen des brevets

(11) **EP 0 816 308 A1**

(12) **EUROPÄISCHE PATENTANMELDUNG**

(43) Veröffentlichungstag:
07.01.1998 Patentblatt 1998/02

(21) Anmeldenummer: 97109740.7

(22) Anmeldetag: 16.06.1997

(51) Int. Cl.$^6$: **C07B 61/00**, C07C 209/60, C07F 9/50, C07F 15/00, B01J 31/24

(84) Benannte Vertragsstaaten:
BE CH DE FR GB IT LI NL

(30) Priorität: 27.06.1996 DE 19625783

(71) Anmelder: BAYER AG
51368 Leverkusen (DE)

(72) Erfinder:
- Driessen, Hölscher, Birgit, Dr.
  52074 Aachen (DE)
- Keim, Wilhelm, Prof.Dr.
  52074 Aachen (DE)
- Prinz, Thomas
  52070 Aachen (DE)
- Traenckner, Hans-Joachim, Dr.
  51467 Bergisch Gladbach (DE)
- Jentsch, Jörg-Dietrich, Dr.
  45468 Mülheim a.d. Ruhr (DE)

(54) **Komplexe, die Tris-(hydroxyalkyl)phosphane als Liganden enthalten, für Telomerisationen, als Katalysatoren und neue Tris-(hydroxyalkyl)-phosphane enthaltende Komplexe**

(57) Ein Verfahren zur Telomerisation von Dienen mit Nucleophilen in einem flüssigen Zweiphasensystem ist dadurch gekennzeichnet, daß man dabei als Katalysatoren Komplexverbindungen einsetzt, die als Zentralatom ein Übergangsmetall und wenigstens ein Tris-(hydroxyalkyl)-phosphan oder -phosphanoxid als Ligand enthalten. Die Verwendung von Verbindungen, die ein Übergangsmetall als Zentralatom und wenigstens ein Tris-(hydroxy-$C_2$-$C_5$-alkyl)-phosphan oder -phosphanoxid als Ligand enthalten als Katalysatoren, neue derartige Verbindungen und wäßrige Lösungen derartiger Verbindungen sind ebenfalls umfaßt.

EP 0 816 308 A1

## Beschreibung

Die vorliegende Erfindung betrifft ein Telomerisationsverfahren, bei dem als Katalysatoren Komplexverbindungen eingesetzt werden, die als Liganden Tris-(hydroxyalkyl)-phosphane enthalten, die generelle Verwendung von solchen Komplexverbindungen als Katalysatoren und neue, als Liganden Tris-(hydroxyalkyl)-phosphane enthaltende Komplexverbindungen.

In der DE-OS 2 601 520 ist die Herstellung von Tris-(hydroxyethyl)-phosphan aus Vinylacetat und Phosphorwasserstoff in Gegenwart eines Radikalinitiators und anschließender saurer Hydrolyse beschrieben. Andere Tris-(hydroxyalkyl)-phosphane können analog dazu erhalten werden, wenn man andere Alkenylacetate als Vinylacetat einsetzt, z.B. Propenyl-, Butenyl- oder Pentenylacetat. Tris-(hydroxyalkyl)-phosphane können gemäß dieser Veröffentlichung als Ausgangsmaterial zur Herstellung flammhemmender Mittel und als Monomer zur Herstellung von Polyurethanharnstoffen verwendet werden.

Das FR-Patent 1 574 239 beschreibt eine ähnliche Synthese von Tris-(hydroxyalkyl)-phosphanen, allerdings unter Einsatz von Alkenolen statt Alkenylacetaten. Als Verwendung für diese Phosphane ist ihr Zusatz zu Dauerwellenmitteln angegeben.

Das US-Patent 3 489 811 beschreibt die Herstellung von im wesentlichen geruchslosem Tris-(hydroxy-3-propyl)-phosphan aus Phosphorwasserstoff durch Umsetzung mit Allylalkohol und die Verwendung dieses Phosphans für kosmetische Zwecke.

Allgemein ist für Hydroxyalkylphosphane festzustellen, daß dafür verschiedene Synthesemöglichkeiten und Verwendungen bekannt sind.

Die Flüssig/Flüssig-Zweiphasentechnik ist ein Verfahren, um die Vorteile homogener und heterogener Katalysatoren zu verbinden. Diese Methode erlaubt es, Übergangsmetalle enthaltende Katalysatoren durch einfache Phasentrennung von den Produkten und gegebenenfalls restlichen Edukten abzutrennen und die Katalysatorphase dann direkt für die nächste Umsetzung wieder zu verwenden (siehe Angew. Chem. 105, 1588 (1993), Adv. Organomet. Chem. 34, 219 (1992)). Auch Telomerisationsreaktionen sind schon in Zweiphasensystemen vorgenommen worden.

Aus der DE-OS 2 733 516 ist die Telomerisation von Dienen mit Nucleophilen in einem Zweiphasensystem bekannt. Bei diesem Verfahren wurde Wasser als Lösungsmittel für den Katalysator benutzt und der Katalysator wasserlöslich gemacht, indem Übergangsmetallverbindungen mit sulfonierten Phosphanen komplexiert wurden. Als Nukleophile wurden Wasser, Alkohole, Phenole, Säuren, Amine, CH-aktive Substanzen und Silanole eingesetzt.

Die US-Patente 4 356 333 und 4 417 079 und die EP-OS 436 226 beschreiben ein Zweiphasensystem für die Telomerisation von Butadien mit Wasser zu Octadienol. Die Reaktion wird dabei in einem Wasser/Sulfolan-Gemisch durchgeführt, aus dem sich die gebildeten Octadienole abscheiden. Der Palladium-Katalysator wird mit Hilfe von monosulfoniertem Triphenylphosphan (TPPMS) in der Sulfolanphase zurückgehalten.

Auch wasserlösliche quartäre Ammoniumphosphane als Liganden für Übergangsmetallkatalysatoren sind in einem Zweiphasensystem für die Telomerisation von Dienen mit Methanol beschrieben worden (J. Mol. Catal. 59, 1 (1990)).

Das FR-Patent 2 693 188 beschreibt die Telomerisation von Saccharose mit Butadien in wäßriger Lösung unter Verwendung eines Katalysatorsystems aus Palladiumacetat und trisulfoniertem Triphenylphosphan (TPPTS). Der Umsatz bezüglich Saccharose beträgt 96 %. Es entstehen verschiedene Octadienylether mit unterschiedlichen Alkylierungsgraden, wobei die Di-, Tri- und Tetraether überwiegen.

Kürzlich ist die Telomerisation von Butadien und Wasser in einem Zweiphasensystem mit einem Trialkylamin als Additiv beschrieben worden (J. Mol. Catal. A: Chemical 97, 29 (1995)). Das Katalysatorsystem besteht dabei aus einem Palladiumsalz und TPPMS oder TPPTS. Das Produktgemisch enthält bis zu 5 Telomerisationsprodukte aus der Gruppe Alkohole, Olefine und Ether.

Zusammenfassend ist zu Telomerisationen mit wasserlöslichen, Phosphane enthaltenden Komplexen als Katalysatoren festzustellen, daß bisher als Liganden für solche Komplexe nur Phosphane in Betracht gezogen wurden, die stark polare Gruppen wie Sulfonat- oder Ammoniumgruppen enthalten. Die bekannten Telomerisationen mit derartigen Katalysatoren verlaufen häufig unselektiv, d.h. sie liefern uneinheitliche Produktgemische.

Aus der Dissertation von M.B. Smith, Universität Bristol (1991), ist Tris-(hydroxymethyl)-phosphan als Ligand für Komplexverbindungen und sind katalytische Eigenschaften solcher Komplexverbindungen bekannt.

Gemäß dem Artikel von P.G. Pringle in I.T. Horvath und F. Joo, Aqueous Organometallic Chemistry and Catalysis, Kluwer Academic Press, S. 114 (1995) wurde Tris-(hydroxy-3-propyl)-phosphan als Ligand für Platin-Komplexe eingesetzt und physikalisch und chemisch charakterisiert. Über katalytischen Effekte solcher Komplexe ist nichts angegeben.

JP 92-149 437 und JP 92-49 538 beschreiben Katalysatoren für Hydroformylierungen, die Rhodium- und/oder Cobaltcarbonyle und Tris-(hydroxymethyl)-phosphan auf einem Träger enthalten.

Zu Komplexen mit Tris-(hydroxyalkyl)-phosphanen als Liganden ist festzustellen, daß nur zwei derartige Komplexverbindungen und nur für eine davon katalytische Eigenschaften bekannt sind.

Die vorliegende Erfindung betrifft zunächst ein Verfahren zur Telomerisation von Dienen mit Nucleophilen in einem flüssigem Zweiphasensystem, das dadurch gekennzeichnet ist, daß man dabei als Katalysatoren Komplexverbindungen einsetzt, die als Zentralatom ein Übergangsmetall und wenigstens ein Tris-(hydroxyalkyl)-phosphan oder -phosphanoxid als Ligand enthalten.

Als Diene kommen z.B. solche in Frage, die 4 bis 12 C-Atome enthalten und gegebenenfalls mit 1 bis 4 $C_1$-$C_4$-Alkylgruppen substituiert sein können. Bevorzugte Diene sind konjugierte Diene wie Isopren, 1,3-Butadien, 1,3-Pentadien, 2,3-Dimethyl-1,3-butadien, 2,4-Hexadien und Myrcen.

Ein besonders bevorzugtes Dien ist 1,3-Butadien.

Als Nucleophile kommen z.B. Wasser, Alkohole, Phenole, Säuren, Ammoniak, Amine, Imine, Kohlendioxid, CH-aktive organische Verbindungen und Silanole in Frage.

Alkohole können beispielsweise 1 bis 12 C-Atome, Phenole beispielsweise 6 bis 20 C-Atome enthalten. Säuren können z.B. organische Säuren mit 1 bis 10 C-Atomen sein, die gesättigt oder ungesättigt und geradkettig, verzweigt oder cyclisch sein und z.B. 1 bis 3 Carboxylatgruppen enthalten können. Amine können z.B. 1 bis 30 C-Atome enthalten und primär oder sekundär sein. Imine können beispielsweise geradkettig sein und 1 bis 10 C-Atome enthalten oder cyclisch sein und 2 bis 5 C-Atome enthalten. Bei den CH-aktiven organischen Verbindungen kann es sich z.B. um Methylencarbonylverbindungen wie Benzylmethylketon, um Nitroalkane wie Nitroethan, um Nitrile wie Ethylcyanoacetat und um Sulfone wie Ethyl-(phenyl-sulfonyl)-acetat handeln. Silanole können z.B. nur Silicium-, Sauerstoff- und Wasserstoffatome, aber gegebenenfalls zusätzlich auch organische Reste mit jeweils z.B. 1 bis 10 C-Atomen enthalten.

Bevorzugte Nucleophile sind Wasser und Ammoniak, besonders bevorzugt ist Ammoniak.

Pro Mol des jeweiligen Diens kann man z.B. 0,1 bis 10 mol eines Nucleophils einsetzen. Vorzugsweise liegt diese Menge bei 0,25 bis 0,5 mol.

Als flüssige Zweiphasensysteme kommen z.B. solche in Betracht, bei denen eine Phase Wasser oder ein polares Lösungsmittel und die andere Phase ein mit der ersten Phase nicht oder nur wenig mischbares Lösungsmittel enthält. Das flüssige Zweiphasensystem kann auch aus einer hydrophilen und einer mit Wasser nicht oder nur wenig mischbaren organischen Phase bestehen. Der Hauptbestandteil der organischen Phase kann ein mit Wasser nicht oder nur wenig mischbares organisches Lösungsmittel sein. In Betracht kommen beispielsweise aliphatische und aromatische Kohlenwasserstoffe, chlorierte aliphatische und aromatische Kohlenwasserstoffe, Ether, tertiäre Amine und Pyrrolidon. Bevorzugte Lösungsmittel sind z.B. Benzol, Toluol und Methylenchlorid. Man kann auch Gemische von mit Wasser nicht oder nur wenig mischbaren organischen Lösungsmitteln einsetzen. Als mit Wasser nicht oder nur wenig mischbare organische Phase kommt auch das jeweils eingesetzte Dien in Frage.

Die hydrophile Phase kann beispielsweise Wasser oder ein hydrophiles organisches Lösungsmittel sein. Hydrophile organische Lösungsmittel sind z.B. $C_1$-$C_6$-Alkohole und Tetramethylensulfon. Als hydrophile Phase wird vorzugsweise Wasser eingesetzt. Man kann auch Gemische einsetzen, z.B. Gemische von Wasser mit $C_1$-$C_6$-Alkoholen oder mit Tetramethylensulfon.

Als Katalysatoren sind solche bevorzugt, die der Formel (I) entsprechen

$$[L^1{}_x L^2{}_y M]_m{}^{p+} [A]_n{}^{q-} \tag{I},$$

in der

$L^1$  für einen Tris-(hydroxy-$C_1$-$C_5$-alkyl)-phosphan oder -phosphanoxid-Liganden,

$L^2$  für einen Liganden aus der Gruppe H, CO, NO, $PF_3$, $H_2O$, S, Halogene, aromatische Liganden, olefinische Liganden und acetylenische Liganden,

M  für ein Metall oder Metallion eines Übergangselementes der Gruppen I, VII oder VIII des Periodensystems der Elemente,

x  für eine ganze Zahl von 1 bis 6 und

y  für Null oder eine ganze Zahl von 1 bis 5 stehen,

wobei die Summe aus x und y höchstens 6 ergibt und

m  für 1, 2 oder 3 und

n, p und q  jeweils für Null, 1, 2 oder 3 stehen, wobei die Beziehung gilt:

$$m \cdot p = n \cdot q \text{ und}$$

A           für ein Anion mit der Ladung q steht.

$L^1$          steht vorzugsweise für ein Tris-(hydroxy-$C_2$-$C_5$-alkyl)-phosphan oder -phosphanoxid, besonders bevorzugt für Tris-(hydroxypropyl)-phosphan oder -phosphanoxid.

$L^2$          steht vorzugsweise für H, CO, NO, Cl, Allyl, Methallyl, Cyclopentadien, Cyclooctadien, Dibenzylidenaceton oder Diphenylacetylen.

M           steht vorzugsweise für Mn, Fe, Co, Ni, Ru, Rh, Pd, Os, Ir oder Pt oder ein Ion dieser Metalle, insbesondere für Nickel, Palladium oder Platin oder ein Ion dieser Metalle. Besonders bevorzugt steht M für Palladium oder $Pd^{2+}$.

x           steht vorzugsweise für eine ganze Zahl von 2 bis 4 und

y           steht vorzugsweise für Null, 1 oder 2,

wobei die Summe von x + y vorzugsweise höchstens 4 beträgt.

M kann in allen für das jeweilige Metall üblichen Oxidationsstufen vorkommen. Bevorzugt sind die Oxidationsstufen Null und +2. Wenn M in einer von Null verschiedenen positiven Oxidationsstufe vorliegt, handelt es sich bei dem $L_x^1 L_y^2 M$-Teil der Verbindung der Formel (I) um ein entsprechend positiv geladenes Komplexion. Die Ladung solcher Komplexionen kann durch beliebige Anionen A kompensiert werden, beispielsweise durch Halogenid-, Carboxylat-, 1,3-Diketonat, Nitrat-, Phosphat-, Sulfat- und/oder Tetrafluoroborat-Ionen. Bevorzugte Anionen A sind Chlorid-, Acetat-, Acetylacetonat- und Nitrationen.

Soweit es sich bei $L^1$ um Tris-(hydroxyalkyl)-phosphane und -phosphanoxide handelt, bei denen die Alkylgruppe 2 oder mehr C-Atome enthält, kann die Hydroxygruppe an der Alkylgruppe an ein beliebiges C-Atom gebunden sein. Vorzugsweise ist die Hydroxygruppe an das endständige C-Atom der Alkylgruppe gebunden, also möglichst weit vom zentralen Phosphoratom entfernt.

Besonders bevorzugte einzelne Komplexverbindungen der Formel (I) sind Palladium(II)-bis[tris-(hydroxy-3-propyl)-phosphan]-acetat, Palladium(II)-tetrakis-[tris-(hydroxy-3-propyl)-phosphan]-acetat, Palladium(0)-dibenzylidenaceton-tetrakis-[tris-(hydroxy-3-propyl)-phosphan], Palladium(O)-tris-[tris-(hydroxy-3-propyl)-phosphan], Palladium(O)-tetrakis-[tris-(hydroxy-3-propyl)-phosphan], die entsprechenden Phosphanoxide und die entsprechenden, zusätzlich Dibenzylidenaceton-Liganden enthaltenden Verbindungen.

Bezogen auf 1 mol Dien kann man z.B. 0,01 bis 0,001 mol Katalysator einsetzen. Vorzugsweise liegt diese Menge bei 0,002 bis 0,005 mol.

Das erfindungsgemäße Telomerisationsverfahren kann man z.B. bei Temperaturen im Bereich von 30 bis 150°C und Drucken im Bereich 1 bis 30 bar durchführen. Vorzugsweise arbeitet man bei 50 bis 120°C und im Autoklaven, unter den sich bei Reaktionstemperatur von selbst ergebenden Drucken.

Die Reaktion ist i.a. nach 1 bis 20 Stunden beendet. Es ist vorteilhaft, während der Reaktion zu rühren oder das Reaktionsgefäß zu schütteln. Das nach der Umsetzung vorliegende Reaktionsgemisch kann man auf einfache Weise aufarbeiten. Beispielsweise kann man so vorgehen, daß man die wäßrige Phase von der organischen Phase trennt, die wäßrige Phase mit einem mit Wasser nicht oder nur wenig mischbaren organischen Lösungsmittel wäscht, vorzugsweise mit dem in der Reaktion eingesetzten organischen Lösungsmittel, die Waschflüssigkeit mit der abgetrennten organischen Phase vereinigt und daraus die erhaltenen Telomerisationsprodukte abtrennt und auftrennt, z.B. durch Destillation oder Kristallisation.

Bei einer bevorzugten Ausführungsform des erfindungsgemäßen Telomerisationsverfahrens legt man den Katalysator und Wasser bei Raumtemperatur in einem Autoklaven vor, fügt das Nucleophil, mit Wasser nicht oder nur wenig mischbares organisches Lösungsmittel und eine abgemessene Menge Dien bei tiefer Temperatur zu und erhitzt den Autoklaven nach dem Verschließen auf Reaktionstemperatur. Nach Beendigung der Reaktion und gegebenenfalls eines Nachrührens bringt man abschließend den Autoklaven auf Raumtemperatur, entspannt ihn und arbeitet das vorliegende Reaktionsgemisch z.B. wie oben beschrieben auf.

Das erfindungsgemäße Telomerisationsverfahren kann man diskontinuierlich oder kontinuierlich durchführen. Es eignet sich besonders zur Herstellung von Octadienylaminen aus Butadien und Ammoniak und von Octadienolen aus Butadien und Wasser.

Die Herstellung erfindungsgemäß einzusetzender Komplexverbindungen kann auf einfache Weise erfolgen. Beispielsweise kann man ein Salz oder eine bekannte Komplexverbindung eines Übergangsmetalles und Wasser vorlegen und diesem Gemisch wenigstens so viel eines Tris-(hydroxyalkyl)-phosphans oder -phosphanoxids zufügen, wie zur Bildung der jeweiligen erfindungsgemäß einzusetzenden Komplexverbindung theoretisch erforderlich ist. Andere Liganden als Tris-(hydroxyalkyl)-phosphane und -phosphanoxide, z.B. solche, die bei Formel (I) mit dem Symbol $L^2$ gekennzeichnet sind, können z.B. zusammen mit dem Übergangsmetallsalz oder der bekannten Übergangsmetallkomplexverbindung oder separat zugegeben werden. Vorzugsweise setzt man das Phosphan oder Phosphanoxid und gegebenenfalls weitere Liganden in der stöchiometrisch erforderlichen Menge ein. Man kann auch mit weniger oder mehr als den stöchiometrisch erforderlichen Mengen an Phosphanen oder Phosphanoxiden und gegebenenfalls weiteren Liganden arbeiten.

Die Wassermenge kann beispielsweise so bemessen werden, daß bezogen auf 1 mmol eingesetztes Übergangsmetallsalz oder 1 mmol eingesetzten Übergangsmetall-Komplex 10 bis 100 ml Wasser zum Einsatz gelangen.

Auf die zuvor beschriebene Weise erhält man eine wäßrige Lösung einer erfindungsgemäß einzusetzenden Komplexverbindung. Diese ist weitgehend frei von gegebenenfalls unerwünschten Verunreinigungen, wenn man mit stöchiometrischen Mengen der Ausgangsmaterialien gearbeitet hat. Häufig ist es nicht erforderlich, erfindungsgemäß einzusetzende Komplexverbindungen aus dieser wäßrigen Lösung zu isolieren. Man kann sie in das erfindungsgemäße Telomerisationsverfahren auch in Form von wäßrigen Lösungen einsetzen, insbesondere in Form solcher wäßriger Lösungen, wie sie bei der hier beschriebenen Herstellung anfallen.

Die vorliegende Erfindung betrifft weiterhin die generelle Verwendung von Komplexverbindungen der Formel

$$[L_x^{1'} L_y^2 M]_m^{p+} [A]_n^{q-} \qquad (I'),$$

in der

$L^{1'}$     für einen Tris-(hydroxy-$C_2$-$C_5$-alkyl)-phosphan- oder -phosphanoxid-Liganden steht,

und die anderen verwendeten Symbole die bei Formel (I) angegebene Bedeutung haben und in der bei Formel (I) angegebenen Weise miteinander verknüpft sind, als Katalysatoren. Vorzugsweise steht $L^{1'}$ für Tris-(hydroxypropyl)-phosphan oder phosphanoxid. Die anderen verwendeten Symbole stehen vorzugsweise und besonders bevorzugt für das, was bei Formel (I) so bezeichnet ist.

Komplexverbindungen der Formel (I') können nicht nur zur Telomerisation von Dienen mit Nucleophilen in flüssigen Zweiphasensystemen eingesetzt werden, sondern beispielsweise auch für beliebige Telomerisationen, für Hydrierungen, für C-C-Verknüpfungen und, in Kombination mit Edelmetallcarbonylen, für Hydroformylierungen.

Die vorliegende Erfindung betrifft weiterhin neue Verbindungen der Formel

$$[L_x^{1''} L_y^2 M]_m^{p+} [A]_n^{q-} \qquad (I''),$$

in der

$L^{1''}$     für einen Tris-(hydroxy-$C_2$-$C_5$-alkyl)-phosphan- oder -phosphanoxid-Liganden mit der Ausnahme von Tris-(hydroxy-3-propyl)-phosphan steht

und die anderen verwendeten Symbole die bei Formel (I) angegebene Bedeutung haben und in der bei Formel (I) angegebenen Weise miteinander verknüpft sind. Vorzugsweise steht $L^{1''}$ für Tris-(hydroxyethyl, hydroxy-2-propyl, hydroxy-4-butyl und hydroxy-5-pentyl)-phosphan oder -phosphanoxid. Die anderen verwendeten Symbole stehen vorzugsweise und besonders bevorzugt für das, was bei Formel (I) so bezeichnet ist.

Weiter oben ist eine Herstellungsweise für Katalysatoren der Formel (I) beschrieben worden, bei der die Katalysatoren in Form einer wäßrigen Lösung anfallen sowie, daß man diese Katalysatoren auch in der Form von wäßrigen Lösungen in das erfindungsgemäße Telomerisationsverfahren einsetzen kann.

Die vorliegende Erfindung betrifft deshalb auch wäßrige Lösungen von Komplexverbindungen, die dadurch gekennzeichnet sind, daß die gelösten Komplexe als Zentralatom ein Übergangsmetall und wenigstens ein Tris-(hydroxy-$C_2$-$C_5$-alkyl)-phosphan oder -phosphanoxid als Ligand enthalten und die Verwendung erfindungsgemäßer wäßriger Lösungen von Komplexverbindungen als Katalysatoren in flüssigen Zweiphasensystemen.

Erfindungsgemäße wäßrige Lösungen enthalten bevorzugt und besonders bevorzugt Komplexverbindungen, die der Formel (I') entsprechen und bevorzugt und besonders bevorzugt Komplex-Verbindungen der Formel (I'), die dort als bevorzugt und besonders bevorzugt beschrieben sind. Erfindungsgemäße wäßrige Lösungen sind i.a. farblos bis gelb/rot gefärbt.

Flüssige Zweiphasensysteme, in denen erfindungsgemäße wäßrige Lösungen von Komplexverbindungen als Katalysator verwendet werden können, bestehen i.a. aus einer wäßrigen Phase und einer Phase, die ein mit Wasser nicht oder nur wenig mischbares organisches Lösungsmittel enthält.

Aus der bei der oben beschriebenen Herstellung von Katalysatoren der Formel (I) anfallenden wäßrigen Lösung können die Verbindungen der Formeln (I), (I') und (I'') gewünschtenfalls auf einfache Weise isoliert werden, z.B. indem man das Wasser abzieht und den verbleibenden Rückstand chromatographisch aufarbeitet.

Das erfindungsgemäße Telomerisationsverfahren und damit auch die Verwendung von Komplexverbindungen der Formel (I') und die neuen Verbindungen der Formel (I'') gestattet die Herstellung von Octadienylaminen aus Butadien und Ammoniak mit guten Ausbeuten und Selektivitäten, wobei sich nach der Reaktion der Katalysator in der einen und die Reaktionsprodukte in der anderen Phase befinden. Dies ermöglicht eine einfache Aufarbeitung und führt zu minimalen bis gar keinen Katalysatorverlusten.

Es ist ausgesprochen überraschend, daß diese Vorteile auftreten, denn aufgrund des Standes der Technik konnte nicht erwartet werden, daß Phosphane und Phosphanoxide mit Hydroxygruppen, die weniger polar sind als Sulfonat- oder Ammoniumgruppen, den Übergangsmetallkomplexen eine ausreichend gute Wasserlöslichkeit und eine ausreichend geringe Löslichkeit in organischen Medien verleihen. Es war vielmehr zu erwarten, daß sich die erfindungsgemäßen Komplexverbindungen in Wasser nicht ausreichend lösen und deshalb nicht als Katalysatoren für flüssige Zweiphasensysteme zu verwenden sind.

## Beispiele

Allgemeines zur Herstellung der Übergangsmetallkomplexe mit Tris-(hydroxyalkyl)-phosphane oder -phosphanoxiden als Ligand.

Zu einem Äquivalent Metallsalz oder Metallkomplex wurden 1 bis 6 Äquivalente in Wasser gelöstes Tris-(hydroxyalkyl)-phosphan oder -phosphanoxid zugefügt. Das Gemisch wurde einige Minuten bei Raumtemperatur gerührt. Dabei ging die ursprünglich meist nicht lösliche Metallverbindung in Lösung. Es entstanden farblose bis gelb/rot gefärbte homogene Lösungen, die in dieser Form zur Katalyse von Telomerisationsreaktionen in flüssigen Zweiphasensystemen eingesetzt wurden.

## Beispiel 1

Eine Katalysatorlösung, hergestellt aus 0,15 mmol Palladium(II)-acetat und 0,3 mmol Tris-(hydroxy-3-propyl)-phosphan in 5 ml Wasser wurde in einem mit Inertgas gefüllten Autoklaven vorgelegt und 20 ml einer 27,13 molaren wäßrigen Ammoniaklösung und 12,5 ml Toluol zugegeben. Der Autoklav wurde gewogen und dann in einer Ethanol/Trockeneis-Mischung gekühlt. Nun wurden 5,8 ml flüssiges Butadien zugesetzt und der Autoklav im Verlauf einer Stunde wieder auf Raumtemperatur gebracht. Er wurde zur genauen Bestimmung der Butadieneinwaage nochmals gewogen und dann in ein auf 80°C vorgeheiztes Ölbad gehängt. Nach Ablauf einer Reaktionszeit von 17 Stunden wurde der Autoklav auf Raumtemperatur abgekühlt, dann entspannt und geöffnet. Der Inhalt wurde in einen Scheidetrichter überführt und die Phasen getrennt. Die wäßrige Phase wurde mit 10 ml Toluol extrahiert. Durch die vereinigten organischen Phasen wurde zur Entfernung restlichen Butadiens Argon geblasen. Abschließend wurde den vereinigten organischen Phasen eine Probe entnommen, über einem Molekularsieb getrocknet und gaschromatografisch untersucht.

Ergebnisse der gaschromatografischen Untersuchung:

Das untersuchte Reaktionsgemisch enthielt als Reaktionsprodukte im wesentlichen Octa-2,7-dienyl-1-amin (1) und Octa-1,7-dienyl-3-amin (2).

| Selektivität zu (1): | 66 % | Umsatz zu (1): | 45 % |
|---|---|---|---|
| Selektivität zu (2): | 18 % | Umsatz zu (2): | 12 % |

Außerdem hatten sich mit einer Selektivität 9 % sekundäre Amine gebildet.
Der Gesamtumsatz zu Telomerisationsprodukten betrug 63 %.

## Beispiel 2

Es wurde verfahren wie in Beispiel 1, jedoch wurden hier 0,6 mmol Tris-(hydroxy-3-propyl)-phosphan eingesetzt.

Ergebnisse der gaschromatografischen Untersuchung:

| Selektivität zu (1): | 54 % | Umsatz zu (1): | 34 % |
|---|---|---|---|
| Selektivität zu (2): | 29 % | Umsatz zu (2): | 18 % |

Außerdem hatten sich mit einer Selektivität von 9 % sekundäre Amine gebildet.
Der Gesamtumsatz zu Telomerisationsprodukten betrug 58 %.

**Beispiel 3**

Es wurde verfahren wie in Beispiel 2, jedoch wurden statt 0,15 mmol Palladium(II)-acetat 0,15 mmol Bis-(dibenzylidenaceton)-palladium verwendet und die Reaktion nach 14,5 Stunden beendet.

Ergebnisse der gaschromatografischen Untersuchung:

| Selektivität zu (1): | 50 % | Umsatz zu (1): | 25 % |
|---|---|---|---|
| Selektivität zu (2): | 28 % | Umsatz zu (2): | 14 % |

Außerdem hatten sich mit einer Selektivität von 4,5 % sekundäre Amine gebildet.
Der Gesamtumsatz zu Telomerisationsprodukten betrug 41,5 %.

**Beispiel 4**

Es wurde verfahren wie in Beispiel 3, jedoch wurden als Phosphan hier 0,6 mmol Tris-(hydroxy-3-propyl)-phosphanoxid eingesetzt.

Ergebnisse der gaschromatografischen Untersuchung:

| Selektivität zu (1): | 55% | Umsatz zu (1): | 2,7 % |
|---|---|---|---|
| Selektivität zu (2): | 1% | Umsatz zu (2): | 0,3 % |

Außerdem hatten sich mit einer Selektivität von 1 % sekundäre Amine gebildet, was sehr vorteilhaft ist.
Der Gesamtumsatz zu Telomerisationsprodukten betrug 4 %.

**Beispiel 5**

0,113 mmol $Pd(OAc)_2$ und 0,339 mmol Tris-(3-hydroxypropyl)phosphan wurden in 30 ml einer 27,15 gew.-%igen, wässrigen $NH_3$-Lösung gelöst und in einen 125 ml Stahlautoklaven gefüllt. Anschließend wurden 38 g Butadien in diesen einkondensiert. Unter intensiver Durchmischung wurde der Autoklav nun 4 Stunden lang bei einer Innentemperatur von 80°C gehalten. Danach wurde er in einem Eisbad auf Raumtemperatur abgekühlt und das überschüssige Butadien über ein Nadelventil aus dem Autoklaven entfernt. Der Inhalt wurde nun mit 10 ml Toluol versetzt und in einen Scheidetrichter überführt. Die organische Phase wurde abgetrennt, die wässrige Phase nochmals mit 10 ml Toluol extrahiert und die vereinigten organischen Phasen über Molekularsieb getrocknet. Es wurde eine Probe dieser Lösung entnommen und gaschromatographisch untersucht.

Ergebnisse der gaschromatographischen Untersuchungen:

Das untersuchte Reaktionsgemisch enthielt als Reaktionsprodukte im wesentlichen Octa-2,7-dienyl-1-amin (1) und Octa-1,7-dienyl-3-amin (2).

7

| Selektivität zu (1): | 53% | Umsatz zu (1): | 0,4% bzgl. Butadien |
|---|---|---|---|
| Selektivität zu (2): | 41% | Umsatz zu (2): | 0,32% bzgl. Butadien |

Gesamtausbeute: 0,8% bzgl. Butadien.

Die Umsätze und Ausbeuten bzgl. Butadien sind niedrig, weil es in großem Überschuß vorlag.

## Beispiel 6

31,0 mg Bis-($\eta^3$-allyl-$\mu$-iodopalladium) wurden in 5 ml einer wässrigen 27,15 gew.-%igen $NH_3$-Lösung gelöst und zu 22 mg Silbertetrafluoroborat, gelöst in 5 ml 27,15 gew.-%iger $NH_3$-Lösung, gegeben. Das ausgefallene Silberiodid wird über Celite® abfiltriert und mit insgesamt 10 ml einer wässrigen 27,15 gew.-%igen $NH_3$-Lösung nachgespült. Diese farblose Lösung wurde in einen 125 ml Stahlautoklaven überführt und anschließend 38 g Butadien einkondensiert. Unter heftigem Rühren wurde der Autoklav auf 80°C aufgeheizt und dann eine Lösung von 47 mg Tris(3-hydroxypropyl)phosphan in 10 ml einer wässrigen 27,15 gew.-%igen $NH_3$-Lösung über einen Tropftrichter zugegeben. Der Autoklav wurde weitere 0,75 Stunden unter heftiger Durchmischung bei 80°C gehalten. Anschließend wurde er in einem Eisbad auf Raumtemperatur abgekühlt. Die weitere Aufarbeitung erfolgt analog zu Beispiel 5.

Ergebnisse der gaschromatographischen Untersuchung:

Das untersuchte Reaktionsgemisch enthielt als Reaktionsprodukte im wesentlichen Octa-2,7-dienyl-1-amin (1) und Octa-1,7-dienyl-3-amin (2).

| Selektivität zu (1): | 47% | Umsatz zu (1): | 0,06% bzgl. Butadien |
|---|---|---|---|
| Selektivität zu (2): | 40% | Umsatz zu (2): | 0.05% bzgl. Butadien |

Gesamtausbeute: 0,12% bzgl. Butadien.

Die Umsätze und Ausbeuten bzgl. Butadien sind niedrig, weil es in großem Überschuß vorlag.

## Beipiel 7

Es wurde verfahren wie in Beipiel 6, jedoch wurden 23,5 mg Tris-(3-hydroxypropyl)phosphan eingesetzt, und die Reaktionszeit betrug 4 Stunden.

Ergebnisse der gaschromatographischen Untersuchung:

Das untersuchte Reaktionsgemisch enthielt als Reaktionsprodukte im wesentlichen Octa-2,7-dienyl-1-amin (1) und Octa-1,7-dienyl-3-amin (2).

| Selektivität zu (1): | 85% | Umsatz zu (1): | 1,53% bzgl. Butadien |
|---|---|---|---|
| Selektivität zu (2): | 9% | Umsatz zu (2): | 0,16% bzgl. Butadien |

Gesamtausbeute: 1,8% bzgl. Butadien.

Die Umsätze und Ausbeuten bzgl. Butadien sind niedrig, weil es in großem Überschuß vorlag.

**Beispiel 8**

Es wurde verfahren wie in Beispiel 6, jedoch betrug die Reaktionszeit 15 Stunden.

Ergebnisse der gaschromatographischen Untersuchung:

Das untersuchte Reaktionsgemisch enthielt als Reaktionsprodukte im wesentlichen Octa-2,7-dienyl-1-amin (1) und Octa-1,7-dienyl-3-amin (2).

| Selektivität zu (1): | 54% | Umsatz zu (1): | 2,7% bzgl. Butadien |
|---|---|---|---|
| Selektivitat zu (2): | 28% | Umsatz zu (2): | 1,4% bzgl. Butadien |

Gesamtausbeute: 5% bzgl. Butadien.

Die Umsätze und Ausbeuten bzgl. Butadien sind niedrig, weil es in großem Überschuß vorlag.

**Beispiel 9**

Es wurde verfahren wie in Beispiel 6, jedoch wurden 23,5 mg Tris-(3-hydroxypropyl)phosphan eingesetzt, und die Reaktionszeit betrug 15 Stunden.

Ergebnisse der gaschromatographischen Untersuchung:

Das untersuchte Reaktionsgemisch enthielt als Reaktionsprodukte im wesentlichen Octa-2,7-dienyl-1-amin (1) und Octa-1,7-dienyl-3-amin (2).

| Selektivität zu (1): | 68% | Umsatz zu (1): | 6,8% bzgl. Butadien |
|---|---|---|---|
| Selektivität zu (2): | 11% | Umsatz zu (2): | 1,1% bzgl. Butadien |

Gesamtausbeute: 10% bzgl. Butadien.

**Patentansprüche**

1. Verfahren zur Telomerisation von Dienen mit Nucleophilen in einem flüssigen Zweiphasensystem, dadurch gekennzeichnet, daß man dabei als Katalysatoren Komplexverbindungen einsetzt, die als Zentralatom ein Übergangsmetall und wenigstens ein Tris-(hydroxyalkyl)-phosphan oder - phosphanoxid als Ligand enthalten.

2. Verfahren nach Anspruch 1, dadurch gekennzeichnet, daß man als Diene solche einsetzt, die 4 bis 12 C-Atome enthalten und gegebenenfalls mit 1 bis 4 $C_1$-$C_4$-Alkylgruppen substituiert sind, als Nucleophile Wasser, Alkohole, Phenole, Säuren, Ammoniak, Amine, Imine, Kohlendioxid, CH-aktive organische Verbindungen oder Silanole einsetzt, als Zweiphasensysteme solche einsetzt, bei denen eine Phase Wasser oder ein polares Lösungsmittel und die andere Phase ein mit der ersten Phase nicht oder nur wenig mischbares Lösungsmittel enthält und als Katalysatoren solche der Formel (I) einsetzt

$$[L^1{}_x L^2{}_y M]_m{}^{p+} [A]_n{}^{q-} \qquad (I),$$

in der

$L^1$ für einen Tris-(hydroxy-$C_1$-$C_5$-alkyl)-phosphan- oder -phosphanoxid-Liganden,

$L^2$ für einen Liganden aus der Gruppe H, CO, NO, $PF_3$, $H_2O$, S, Halogene, aromatische Liganden, olefinische Liganden und acetylenische Liganden,

M für ein Metall oder Metallion eines Übergangselementes der Gruppen I, VII oder VIII des Periodensystems der Elemente,

x für eine ganze Zahl von 1 bis 6 und

y für Null oder eine ganze Zahl von 1 bis 5 stehen,

wobei die Summe aus x und y höchstens 6 ergibt und

m für 1, 2 oder 3 und

n, p und q jeweils für Null, 1, 2 oder 3 stehen, wobei die Beziehung gilt:

$$m \cdot p = n \cdot q \text{ und}$$

A für ein Anion mit der Ladung q steht.

3. Verfahren nach Ansprüchen 1 und 2, dadurch gekennzeichnet, daß man pro mol Dien 0,01 bis 0,001 mol Katalysator einsetzt und bei 30 bis 150°C und 1 bis 30 bar arbeitet.

4. Verwendung von Komplexverbindungen der Formel

$$[L_x^{1'} L_y^2 M]_m^{p+} [A]_n^{q-} \qquad (I'),$$

in der

$L^{1'}$ für einen Tris-(hydroxy-$C_2$-$C_5$-alkyl)-phosphan- oder -phosphanoxid-Liganden steht, und die anderen verwendeten Symbole die bei Formel (I) in Anspruch 1 angegebene Bedeutung haben und in der bei Formel (I) angegebenen Weise miteinander verknüpft sind,

als Katalysatoren.

5. Verbindungen der Formel

$$[L_x^{1''} L_y^2 M]_m^{p+} [A]_n^{q-} \qquad (I''),$$

in der

$L^{1''}$ für einen Tris-(hydroxy-$C_2$-$C_5$-alkyl)-phosphan- oder -phosphanoxid-Liganden mit der Ausnahme von Tris-(hydroxy-3-propyl)-phosphan steht

und die anderen verwendeten Symbole die bei Formel (I) in Anspruch 2 angegebene Bedeutung haben und in der dort angegebenen Weise miteinander verknüpft sind.

6. Wäßrige Lösungen von Komplexverbindungen, dadurch gekennzeichnet, daß die gelösten Komplexe des Zentralatom ein Übergangsmetall und wenigstens ein Tris-(hydroxy-$C_2$-$C_5$-alkyl)-phosphan oder -phosphanoxid als Ligand enthalten.

7. Verwendung von wäßrigen Lösungen von Komplexverbindungen des Anspruchs 6 als Katalysatoren in flüssigen Zweiphasensystemen.

**Europäisches Patentamt**

# EUROPÄISCHER RECHERCHENBERICHT

Nummer der Anmeldung

EP 97 10 9740

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,A | DE 27 33 516 A (RHONE-POULENC INDUSTRIES) * Ansprüche; Beispiele * --- | 1-7 | C07B61/00 C07C209/60 C07F9/50 |
| D,A | EP 0 436 226 A (KURARAY) * Ansprüche; Beispiele * --- | 1-7 | C07F15/00 B01J31/24 |
| A | P. A. T. HOYE: "Hydrophosphination of formaldehyde catalysed by tris(hydroxymethyl)phosphine complexes of platinum, palladium or nickel" JOURNAL OF THE CHEMICAL SOCIETY, DALTON TRANSACTIONS, Nr. 2, 1993, LETCHWORTH GB, Seiten 269-274, XP002042501 * Zusammenfassung * --- | 4-7 | |
| P,X | J CERMAK: "Nickel(0) and palladium(0) complexes with 1,3,5-triaza-7-phosphaadamantane. Catalysis of buta-1,3-diene oligomerization or telomerization in an aqueous biphasic system" COLLECTION OF CZECHOSLOVAK CHEMICAL COMMUNICATIONS., Bd. 62, Nr. 2, Februar 1997, PRAGUE CS, Seiten 355-363, XP002042502 * das ganze Dokument * --- | 1-3 | |
| P,X | WO 96 30056 A (CURATORS OF THE UNIVERSITY OF MISSOURI) * Ansprüche 1,5,6; Beispiele 1,2 * --- | 5,6 | |
| P,X | EP 0 773 211 A (BAYER) * Seite 3, Zeile 49 - Seite 4, Zeile 3 * ----- | 1-3 | |

RECHERCHIERTE SACHGEBIETE (Int.Cl.6)

C07B
C07C
C07F

Der vorliegende Recherchenbericht wurde für alle Patentansprüche erstellt

| Recherchenort | Abschlußdatum der Recherche | Prüfer |
|---|---|---|
| DEN HAAG | 6.Oktober 1997 | Wright, M |

EPO FORM 1503 03.82 (P04C03)